# FASCICULE DE BREVET EUROPEEN

(11) **EP 1 727 563 B1**
(45) Date de publication et mention de la délivrance du brevet: **02.05.2012**
(21) Numéro de dépôt: 05744568.6
(22) Date de dépôt: 25.03.2005
(51) Int. Cl.: A61K 39/21, C07K 16/28

(54) **PROCEDE POUR AMPLIFIER L'ACTIVITE DE VACCINS THERAPEUTIQUES**
VERFAHREN ZUR VERSTÄRKUNG DER AKTIVITÄT EINES THERAPEUTISCHEN IMPFSTOFFS
METHOD FOR AMPLIFYING THERAPEUTIC VACCINE ACTIVITY

(30) Priorité: 26.03.2004 FR 0403178
(43) Date de publication de la demande: 06.12.2006
(73) Titulaire: Biovaxim Limited, London EC1V 9EE (GB)
(72) Inventeur: ANDRIEU, Jean-Marie, F-75005 Paris (FR); LU, Louis, F-75015 Paris (FR)
(74) Mandataire: Novagraaf Technologies
(86) Numéro de dépôt international: PCT/FR2005/000729
(87) Numéro de publication internationale: WO 2005/092373

(56) Documents cités:
- US-A1- 2003 103 971
- DATABASE BIOSIS [Online] BIOSCIENCES INFORMATION SERVICE, PHILADELPHIA, PA, US; 16 novembre 2003 (2003-11-16), WILSON WYNDHAM H ET AL: "Idiotype vaccine and dose-adjusted EPOCH-rituximab treatment in untreated mantle cell lymphoma: Preliminary report on clinical outcome and analysis of immune response." XP002313046 Database accession no. PREV200400172557
- DATABASE BIOSIS [Online] BIOSCIENCES INFORMATION SERVICE, PHILADELPHIA, PA, US; 16 novembre 2003 (2003-11-16), WENG WEN-KAI ET AL: "Analysis of tumor-specific T cell immune response in follicular non-Hodgkin's lymphoma patients treated with rituximab." XP002313047 Database accession no. PREV200400133925
- DATABASE BIOSIS [Online] BIOSCIENCES INFORMATION SERVICE, PHILADELPHIA, PA, US; 1 septembre 2004 (2004-09-01), PERMAR SALLIE R ET AL: "Limited contribution of humoral immunity to the clearance of measles viremia in rhesus monkeys" XP002313048 Database accession no. PREV200400408518

## Description

L'invention se place dans le domaine de l'immunologie, plus spécifiquement dans celui de l'immunothérapie active spécifique aussi appelé vaccin thérapeutique.

Elle est en rapport avec l'utilisation d'un ou plusieurs composés déplétant les lymphocytes B du système immunitaire. Ces composés sont destinés à être administrés à un patient au moment d'une vaccination, particulièrement une vaccination thérapeutique, contre une tumeur et/ou une infection chronique virale, parasitaire ou à germe intracellulaire dans le but d'amplifier et/ou de prolonger l'activité cytotoxique des lymphocytes T à l'égard des cellules tumorales ou des cellules infectées par un virus, un parasite ou un germe intracellulaires.

Le système immunitaire est chargé de détruire (élimination) ou d'empêcher la prolifération (maintenir dans l'organisme à très basse concentration) les microbes, virus et parasites qui y ont pénétré. Le système immunitaire est principalement formé des organes lymphoïdes secondaires, du thymus, de la moelle osseuse et d'un réseau de cellules placées près des revêtements cutanés et muqueux. Les organes lymphoïdes secondaires principaux sont les ganglions externes situés au niveau du cou, des aisselles, des aines et les ganglions internes situés au niveau du thorax et de l'abdomen. Les plaques de Peyer situés le long du tube digestif sont aussi des formations très importantes du système immunitaire de même que la rate.

Le système immunitaire est formé de plusieurs types de cellules qui ont chacune des spécificités particulières. Les cellules dendritiques issues des monocytes du sang, sont chargées d'ingérer de minimes fractions des pathogène (virus, bactéries, parasites), en général dans la région où ils ont pénétré l'organisme, puis de les couper en morceau pour les présenter à leur surface sous forme de peptides (fragments de protéines) appelés antigènes. Dans le même temps, les cellules dendritiques migrent vers les différentes composantes du système immunitaire.

Les cellules immunitaires les plus nombreuses sont appelées les lymphocytes; elles circulent dans le sang, mais la majorité des lymphocytes se trouvent dans la moelle et les organes lymphoïdes. Au sein de ces organes, ils sont capables de reconnaître les antigènes présentés à la surface des cellules dendritiques.

Il existe plusieurs types de lymphocytes :
- Les lymphocytes B se multiplient et se différencient au contact d'une cellule dendritique présentant l'antigène. Ces lymphocytes B se différencient au cours de leur multiplication.Ils prennent alors le nom de plasmocytes et siègent principalement dans la moelle ; ils produisent des anticorps spécifiques des peptides antigéniques qui ont été présentés aux lymphocytes B par les cellules dendritiques (ou d'autres types cellulaires capables de présenter des antigènes). Les anticorps sont des protéines qui circulent dans les vaisseaux et les espaces extracellulaires ; ils sont capables de se fixer sur l'antigène en cause et de préparer ainsi la destruction des cellules porteuses de cet antigène. Ces anticorps sont aussi capables de neutraliser l'activité biologique de micro-organismes qui circuleraient dans le sang ou les milieux extra cellulaires et de préparer leur destruction.
- Les lymphocytes T CD8 sont principalement les lymphocytes cytotoxiques. Après multiplication puis maturation, au sein du système immunitaire et particulièrement au sein des organes lymphoïdes, au contact de la cellule dendritique porteuse d'antigène, ces lymphocytes T CD8 acquièrent la capacité de détruire toutes les cellules portant l'antigène avec lesquelles elles rentrent en contact.
- Une troisième catégorie de lymphocytes, les lymphocytes T CD4, est absolument indispensable à la prolifération et à la maturation des lymphocytes B (qui vont produire des anticorps) et des lymphocytes T CD8 (qui vont produire des cellules cytotoxiques). Ces lymphocytes T CD4 sont aussi spécifiques de chaque type d'antigène ; après être entrés au contact de la cellule dendritique au sein des organes lymphoïdes, ils ont la capacité particulière de favoriser la multiplication et la maturation des lymphocytes CD8 (il s'agit alors particulièrement de lymphocytes T CD4 , helpers de type 1 (TH1)) ou de favoriser la multiplication et la maturation de lymphocytes B spécifiques de tel ou tel antigène (il s'agit alors de lymphocytes T CD4 TH2).

Le système immunitaire est ainsi particulièrement efficace pour contrôler ou au mieux pour éliminer de l'organisme des cellules infectées par différents types de micro-organismes tels que virus, microbe, parasites. Certains types de pathogènes nécessitent majoritairement ou exclusivement la mise en marche de lymphocytes B à l'origine de la production d'anticorps ; on dit qu'ils mettent en jeu l'immunité humorale. A l'inverse, certains virus, parasites ou microbes situés à l'intérieur des cellules, nécessitent préférentiellement la mise en marche de lymphocytes TCD8 capables de détruire ces cellules infectées. Ces lymphocytes CD8 sont responsables de ce que l'on nomme l'immunité cellulaire.

Une vaccination thérapeutique consiste en l'administration d'antigènes spécifiques de tumeurs ou de microbes, virus ou parasites. Ces antigènes spécifiques peuvent être administrés au patient sous forme de cellules infectées ou tumorales, préalablement inactivées (par un moyen physique ou chimique). Ils peuvent aussi être administrés sous forme de protéines ou de peptides mais aussi d'ADN ou d'ARN spécifiques des protéines ou des peptides en cause. Ces ADN ou ces ARN peuvent être eux-mêmes libres ou introduits au sein de vecteurs ADN ou ARN viraux ou non viraux. Quels que soient le type et la forme de la préparation administrée, le plus souvent par voies sous-cutanées (les voies intramusculaires et intraveineuses peuvent aussi être utiles, de même que la voie *per os*), l'objectif final est que cette préparation soit finalement transformée en peptides présentés à la surface des cellules dendritiques (ou d'autres types de cellules capables de présenter l'antigène).

Le succès d'une vaccination, quelle que soit la forme de la préparation antigénique, nécessite le plus souvent, l'utilisation concomitante d'adjuvants d'origine minérale, chimique ou de composés biologique d'origine naturelle.

La préparation antigénique (cellule tumorale ou infectée inactivée, protéines, peptide, ADN, ARN) peut aussi être administré *in vitro,* à des cellules dendritiques préparées *ex vivo* à partir de cellules sanguines grâce à des cytokines. Ces cellules dendritiques sont modifiées par l'action des cytokines pour exprimer à leur surface des antigènes (peptides) spécifiques. Une fois qu'elles ont été mises en contact de façon appropriée avec la préparation antigénique, ces cellules dendritiques chargées *ex vivo* en antigènes, sont administrées au patient, le plus souvent par injection sous-cutanée. Ces cellules migrent vers les organes lymphoïdes et provoquent la prolifération et la différenciation des lymphocytes T8 cytotoxiques à l'égard de cellules tumorales ou infectées (qui sont porteuses des antigènes en cause) et/ou à l'égard des lymphocytes B qui, se transformant en plasmocytes producteurs d'anticorps dirigés contre les peptides antigéniques portés par les cellules tumorales ou les cellules infectées, peuvent permettre de faciliter la lyse de ces cellules.

On peut attendre du principe de vaccination thérapeutique qu'il favorise le contrôle ou même l'éradication d'infections chroniques ou de tumeurs. Il demeure que les résultats cliniques de ces vaccins thérapeutiques contre différents types de cancers ou contre des infections chroniques sont très médiocres.

On comprend de ce qui précède, l'importance aussi bien des lymphocytes T cytotoxiques que celles des lymphocytes B dans la réaction immunitaire et particulièrement dans la réussite potentielle de vaccins thérapeutiques. Selon les connaissances actuelles, la présence et l'activité des lymphocytes B apparaît comme essentielle pour obtenir une réaction immunitaire satisfaisante.

Or, de manière surprenante et inattendue, les inventeurs ont maintenant constaté qu'un composé déplétant ou inhibant les lymphocytes B naïfs des organes lymphoïdes, est capable d'amplifier la réaction immunitaire de type cellulaire.

En effet, les inventeurs ont observé que l'administration d'un composé déplétant les lymphocytes B naïfs, permettait d'augmenter la réponse cellulaire T spécifique à un vaccin thérapeutique formé d'un virus de l'immunodéficience du singe (VIS) inactivé par méthode chimique et d'un adjuvant (adjuvant incomplet de Freud) et administré par voie sous-cutanée à des singes infectés par le virus de l'immunodéficience du singe.

Ce résultat surprenant va à l'encontre des connaissances généralement admises concernant la réaction immunitaire telles qu'elles ont été brièvement exposées précédemment. Il apparaît en effet que la déplétion des lymphocytes B lorsqu'elle est associée à une vaccination visant à stimuler l'immunité cellulaire, non seulement ne semble pas délétère, mais semble favoriser l'efficacité de la réaction de l'immunité cellulaire.

Ainsi les inventeurs proposent à titre de premier objet de l'invention, l'utilisation d'un anticorps dirigé contre l'antigène transmembranaire CD20 des lymphocytes pré-B ou B matures en combinaison avec un vaccin thérapeutique comprenant un virus de l'immunodéficience humaine (VIH) inactivé, dans la préparation d'une composition pour le traitement d'une infection par le virus de l'immunodéficience humaine (VIH).

Préférentiellement, l'anticorps est un anticorps monoclonal dirigé contre l'antigène transmembranaire CD20 des lymphocytes pré-B ou B matures.

Ledit anticorps peut être un anticorps naturel, ou obtenu par génie génétique. L'anticorps peut être d'origine humaine, ou de tout autre mammifère comme par exemple murin ou encore produit par génie génétique, comme par exemple dans des microorganismes, ou encore par synthèse chimique.

L'anticorps peut être ou ne pas être humanisé. Il peut être un anticorps chimère ou recombiné. Particulièrement, l'anticorps peut être l'anticorps monoclonal vendu sous la dénomination RITUXIMAB^{®}. Il s'agit alors d'un anticorps chimérique murin/humain obtenu par génie génétique ; il s'agit d'une immunoglobuline glycosylée associant d'une part les régions constantes d'une IgG1 humaine et d'autre part les régions variables des chaînes légères et lourdes d'origine murine. Cet anticorps est produit par une culture de cellules de mammifères (ovaires de hamster chinois) et purifié par chromatographie d'affinité et d'échange d'ions, comportant des procédés d'inactivation et d'élimination virales spécifiques.

Cet anticorps, particulièrement son fragment Fab, se lie spécifiquement à l'antigène transmembranaire CD20 des lymphocytes B. Cet antigène ne s'internalise pas lors de la liaison à l'anticorps et il n'est pas libéré de la surface cellulaire. Le CD20 ne circule pas sous forme libre dans le plasma et n'entre donc pas en compétition pour la liaison à l'anticorps.

Une fois lié à l'antigène CD20 des lymphocytes B, le complexe formé entre l'anticorps, ou le fragment Fab de celui-ci, et l'antigène CD20, génère des fonctions d'effecteur immunitaire qui entraînent la lyse de ces lymphocytes par l'intermédiaire du fragment Fc. Les mécanismes possibles de la lyse cellulaire sont une cytotoxicité dépendante du complément (CDC), faisant intervenir la liaison du fragment Clq, et une cytotoxicité cellulaire dépendante des anticorps (ADCC), passant par un ou plusieurs des récepteurs Fc gamma de la surface des granulocytes, des macrophages et des cellules NK.

Ainsi, selon l'invention, l'anticorps dirigé contre l'antigène transmembranaire CD20 des lymphocytes pré-B ou B matures peut être un fragment Fab dudit anticorps.

Selon l'invention, la composition dans la préparation de laquelle l'anticorps dirigé contre l'antigène transmembranaire CD20 des lymphocytes pré-B ou B matures est utilisé, peut être administrée par tout moyen connu, antérieurement, concomitamment ou postérieurement à une vaccination, particulièrement une vaccination thérapeutique contre une tumeur et/ou contre une infection chronique virale, parasitaire ou à germe intra cellulaire., L'administration de la composition selon l'invention peut être réalisée par tout moyen adéquat connu. On citera par exemple l'injection, particulièrement l'injection sous-cutanée ou intraveineuse ou intramusculaire, ou encore l'administration par voie orale. Préférentiellement, l'administration est réalisée par une injection intraveineuse.

La composition de l'invention, peut comprendre tout support connu, biologiquement compatible pour une administration à un patient. On peut citer à titre d'exemple, l'eau déminéralisée stérile, le sérum physiologique ou encore une solution pour perfusion.

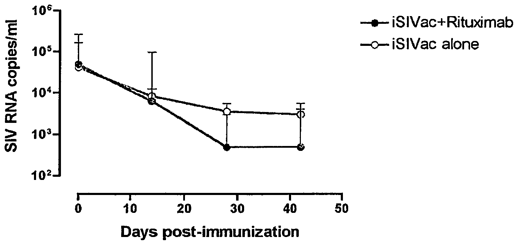

La figure 1 représente le résultat obtenu par une vaccination thérapeutique pratiquée sur des singes infectés par un virus de l'immunodéficience du singe (VIS) et traités ou non selon l'invention avec un anticorps monoclonal dirigé contre l'antigène transmembranaire CD20 des lymphocytes B (RITUXIMAB^{®}). Les courbes représentent le nombre de copies d'ARN du virus VIS par millilitre de plasma des singes infectés par le VIS puis ayant reçu un an après l'infection un vaccin thérapeutique composé de virus VIS inactivés et d'adjuvant, et traités ou non au RITUXIMAB^{®}, en fonction du nombre de jours après la vaccination thérapeutique.

Dans cette figure sont représentés les résultats obtenus avec :
○ : un vaccin VIS inactivé + adjuvant ;
● : un vaccin VIS inactivé + adjuvant précédé (J-3) et suivi (j + 4 et j + 11) d'une administration de RITUXIMAB^{®}.

D'autres caractéristiques de l'invention apparaîtront dans l'exemple de réalisation de l'invention, ainsi que dans la figure, sans pour autant que ceux-ci ne constituent une quelconque limitation de l'invention.

EXEMPLE : Mesure de l'effet d'un composé déplétant les lymphocytes B sur la réponse des cellules T après vaccination thérapeutique contre un virus
□ Matériels et Méthodes :
   - Le projet de recherche a été approuvé par le comité des études animales de l'Institut de Médecine Tropicale de Guangzhou, Chine.
   - Préparation du virus inactivé : le virus VISmac251 a été inactivé par traitement à l'aldrithiol-2 (AT-2) comme décrit précédemment (Lu, W. et coll., J. Virol., 75 : 8949-8956, 2001). Le virus VIS-AT-2 inactivé a été concentré par ultracentrifugation pour obtenir une concentration finale de 2.10¹⁰ particules virales /ml, et a ensuite été congelé à -80°C pour conservation jusqu'à son utilisation.
   - Animaux : 8 macaques adultes sains, rhésus "colony-bred" proviennent du Shunde Experimental Animal Center (Guangdong, China). Les animaux ont été infectés avec le virus VISmac251, comme décrit précédemment (Lu, W. et coll., Nat. Med. 9 : 27-32, 2003), une année avant la vaccination thérapeutique.
   - Préparation du vaccin thérapeutique : Le virus VIS-AT-2 inactivé a été décongelé à température ambiante. 10¹⁰ particules virales (0,5ml) ont alors été mélangées à 0,5 ml d'adjuvant incomplet de Freund (Sigma-Aldrich Chimie Sarl, Saint Quentin Fallavier, France) pour donner 1 ml de vaccin VIS-AT-2 inactivé. Ce mélange a été utilisé pour immuniser les animaux.
   - Vaccination : Les 8 animaux ont reçu 1 injection sous-cutanée de 0,25 ml à la racine des 4 membres (soit au total 1 ml) de vaccin VIS-AT-2 inactivé 4 d'entre eux ont reçu du RITUXIMAB^{®} par voie intraveineuse, à raison de 10 mg/kg, 3 jours avant la vaccination thérapeutique, puis 4 jours et 11 jours après ladite vaccination thérapeutique.
   - Mesures virologiques et immunologiques : La mesure de la charge virale et de la cytolyse virospécifique ont été réalisées régulièrement toutes les 2 semaines, comme décrit précédemment (Lu, W. et coll., J. Virol., 75 : 8949-8956, 2001 ; Lu, W. et coll., Nat. Med. : 1081-1085, 1999) sans modification. La réponse des cellules CD4⁺ Th1 et des cellules-T mémoires CD8⁺ a été mesurée par le test en spot de la sécrétion de l'interféron-γ à l'aide du kit ELISPOT de R&D Systems Europe (Lille, France) selon les recommandations du fournisseur.
   - Analyses statistiques : Les tests de Mann-Whitney ou de Wilcoxon ont été utilisés pour comparer les données avant et après immunisation.
□ Résultats :
   Mesure de l'effet du composé déplétant les lymphocytes B :
      4 semaines après la vaccination thérapeutique, la quantité d'ARN du virus de l'immunodéficience du singe (VIS) contenue dans le plasma des singes a diminué de 100 fois chez les animaux vaccinés et traités au RITUXIMAB^{®} et de 10 fois chez les animaux vaccinés mais non traités au RITUXIMAB^{®}. La figure 1 présente ces résultats.
□ Conclusion
   Il apparaît que la déplétion temporaire, ou l'inhibition des lymphocytes B naïfs est un outil puissant dans la promotion de la réponse cytotoxique antigène spécifique des cellules T au cours de l'immunisation contre des virus ou des tumeurs.

## Revendications

1. Utilisation d'un anticorps dirigé contre l'antigène transmembranaire CD20 des lymphocytes pré-B ou B matures en combinaison avec un vaccin comprenant un virus de l'immunodéficience humaine (VIH) inactivé, dans la préparation d'une composition pour le traitement thérapeutique d'une infection par le virus de l'immunodéficience humaine (VIH).

2. Utilisation selon la revendication 1, **caractérisée en ce que** l'anticorps dirigé contre l'antigène transmembranaire CD20 des lymphocytes pré-B ou B matures est un anticorps polyclonal ou monoclonal, ou un fragment Fab de l'anticorps.

3. Utilisation selon l'une quelconque des revendications 1 ou 2, **caractérisée en ce que** l'anticorps est un anticorps monoclonal.

4. Utilisation selon l'une quelconque des revendications 1 à 3, **caractérisée en ce que** l'anticorps est un anticorps chimérique murin/humain obtenu par génie génétique.

5. Utilisation selon l'une quelconque des revendications 1 à 4, **caractérisée en ce que** l'anticorps est administré antérieurement et/ou concomitamment et/ou postérieurement au vaccin comprenant un virus de l'immunodéficience humaine (VIH) inactivé.

## Claims

1. Use of an antibody directed against transmembrane antigen CD20 of the pre-B or mature B lymphocytes in combination with a vaccine comprising an inactivated human immunodeficiency virus (HIV), in the preparation of a composition for the therapeutic treatment of an infection by the human immunodeficiency virus (HIV).

2. Use according to claim 1, **characterized in that** the antibody directed against transmembrane antigen CD20 of the pre-B or mature B lymphocytes is a polyclonal or monoclonal antibody, or a Fab fragment of the antibody.

3. Use according to any one of claim 1 or 2, **characterized in that** the antibody is a monoclonal antibody.

4. Use according to any one of claims 1 to 3, **characterized in that** the antibody is a murine/human chimeric antibody obtained by genetic engineering.

5. Use according to any one of claims 1 to 4, **characterized in that** the antibody is administered prior to and/or concomitant with and/or subsequent to a vaccine comprising an inactivated human immunodeficiency virus (HIV).

## Patentansprüche

1. Verwendung eines gegen das Transmembran-Antigen CD20 der Prä-B oder reifen B-Lymphozyten gerichteten Antikörpers in Kombination mit einer Impfung, die ein inaktiviertes Humanes Immundefizienz Virus (HIV) enthält zur Zubereitung einer Zusammensetzung zur therapeutischen Behandlung einer Infektion mit dem Humanen Immundefizienz-Virus (HIV).

2. Verwerdung nach Anspruch 1, **dadurch gekennzeichnet, dass** der gegen das Transmembran-Antigen CD20 der prä-B oder reifen B-Lymphozyten gerichtete Antikörper ein polyklonaler oder monoklonaler Antikörper oder ein Fab-Fragment des Antikörpers ist.

3. Verwerdung nach Anspruch 1 oder 2, **dadurch gekennzeichnet, dass** der Antikörper ein monoklonaler Antikörper ist.

4. Verwerdung nach einem der Ansprüche 1 bis 3, **dadurch gekennzeichnet, dass** der Antikörper ein murin/human-chimärer Antikörper ist, der gentechnisch erhalten wurde.

5. Verwerdung nach einem der Ansprüche 1 bis 4, **dadurch gekennzeichnet, dass** der Antikörper vor und/oder gleichzeitig mit und/oder nach einer Impfung, die ein inaktiviertes Humanes Immundefizienz-Virus (HIV) umfasst, verabreicht wird.
